# FASCICULE DE BREVET EUROPEEN

(11) **EP 0 782 881 B1**
(45) Date de publication et mention de la délivrance du brevet: **10.06.1998**
(21) Numéro de dépôt: 96402684.3
(22) Date de dépôt: 10.12.1996
(51) Int. Cl.: B01F 3/12, A61K 7/00

(54) **Procédé de dispersion par homogénéisation haute pression de charges pulvérulentes dans un support se présentant sous la forme d'une dispersion huile-dans-eau ou eau-dans-huile, compositions obtenues et utilisations**
Verfahren zum Dispergieren durch Hochdruckhomogenisieren von pulverförmigen Produkten in einem Mittel, das aus einer Öl-Wasser oder einer Wasser-Öl Dispersion besteht, erhaltene Zusammensetzungen und deren Anwendung
Process for dispersing through high pressure homogenisation of pulverulent charges in a medium consisting of an oil-water or a water-oil dispersion, compositions obtained and their utilisation

(30) Priorité: 03.01.1996 FR 9600029
(43) Date de publication de la demande: 09.07.1997
(73) Titulaire: L'OREAL, 75008 Paris (FR)
(72) Inventeur: Le Royer, Isabelle, 78350 Jouy en Josas (FR); Thibaut, Catherine, 75013 Paris (FR)
(74) Mandataire: Miszputen, Laurent

(56) Documents cités:
- WO-A-95/05144
- GB-A- 1 050 985
- PATENT ABSTRACTS OF JAPAN vol. 008, no. 199 (C-242), 12 Septembre 1984 & JP 59 091123 A (TOYO BOSEKI KK), 25 Mai 1984,
- PATENT ABSTRACTS OF JAPAN vol. 013, no. 241 (C-604), 6 Juin 1989 & JP 01 051128 A (TOYOBO CO LTD;OTHERS: 01), 27 Février 1989,
- PATENT ABSTRACTS OF JAPAN vol. 008, no. 193 (C-241), 5 Septembre 1984 & JP 59 086640 A (DAICEL KAGAKU KOGYO KK), 18 Mai 1984,
- PATENT ABSTRACTS OF JAPAN vol. 008, no. 193 (C-241), 5 Septembre 1984 & JP 59 084938 A (DAICEL KAGAKU KOGYO KK), 16 Mai 1984,

## Description

La présente demande concerne un procédé de dispersion selon la revendication 1 d'au moins une charge organique et/ou d'au moins une charge minérale pulvérulente dans un support, ainsi que les compositions cosmétiques ou dermatologiques selon la revendication 18.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions destinées à la photoprotection (UV-A et/ou UV-B) de la peau et/ou du cuir chevelu ont été proposées à ce jour et, dans ce domaine, on observe que l'utilisation de nanopigments minéraux (c'est à dire de pigments dont la taille moyenne des particules primaires n'excède généralement pas 100 nm) à base d'oxydes métalliques, et en particulier d'oxyde de titane, devient de plus en plus fréquente, compte tenu du fait que ces dernières substances, lorsqu'elles sont associées avec des filtres UV classiques (principalement des composés organiques capables d'absorber les rayonnements nocifs) permettent d'obtenir des indices de protection très élevés.

Pour diverses raisons liées en particulier à un meilleur confort d'utilisation (douceur, émollience et autres), les compositions photoprotectrices actuelles se présentent le plus souvent sous la forme d'une dispersion de type huile-dans-eau (c'est à dire un support constitué d'une phase continue dispersante aqueuse et d'une phase discontinue dispersée huileuse) ou de type eau-dans-huile (c'est à dire un support constitué d'une phase continue dispersante grasse et d'une phase discontinue dispersée aqueuse) dans laquelle on a introduit, à des concentrations diverses, les nanopigments susmentionnés, le cas échéant en association avec d'autres filtres UV classiques, et qui peuvent être présents tant dans la phase aqueuse de la dispersion que dans sa phase huileuse (encore appelée phase grasse).

L'un des inconvénients majeurs des compositions connues à ce jour et appartenant au type ci-dessus et plus particulièrement de celles contenant des nanopigments d'oxyde de titane TiO₂, est que, une fois appliquées sur la peau sous la forme d'un film, elles engendrent sur cette dernière un effet blanchissant et un toucher rêche qui sont cosmétiquement indésirables et généralement peu appréciés des utilisateurs. Ces effets sont essentiellement dus à la présence d'aggrégats de taille plus ou moins élevée formés par les nanopigments généralement sous forme de poudre.

Une autre difficulté réside par ailleurs dans le fait que les dispersions huile-dans-eau ou eau-dans-huile classiques à base de nanopigments protecteurs conduisent, après application sur la peau, à une distribution irrégulière, non homogène, voire grossière, desdits nanopigments sur cette peau, ce qui peut nuire à la qualité de l'effet de photoprotection global recherché. Cette mauvaise répartition des nanopigments que l'on constate à la surface de la peau est souvent liée au fait qu'il existe, au niveau de la dispersion initiale même (avant application), un manque substantiel d'homogénéité (mauvaise dispersion du pigment dans son support).

La demanderesse a découvert de manière surprenante un nouveau procédé de dispersion de charges organiques et/ou de charges minérales pulvérulentes dans un support constitué d'au moins une phase grasse permettant d'obtenir de nouvelles dispersions de ces charges pulvérulentes subtantiellement plus fines et plus homogènes que les formulations du même type obtenues par des moyens classiques de dispersion de charges (agitation par cisaillement avec un dispositif du type Moritz).

En particulier, les dispersions huile-dans-eau ou eau-dans-huile contenant comme charges pulvérulentes des nanopigments minéraux à base d'oxydes métalliques obtenues selon le procédé de l'invention peuvent constituer de nouvelles compositions cosmétiques ou dermatologiques ne conduisant pas à des phénomènes de blanchissement sur la peau à l'application ou du moins conduisant à un effet blanchissant réduit de manière significative. Elles présentent en outre des propriétés cosmétiques sensiblement améliorées notamment au niveau de la douceur au toucher par rapport aux dispersions huile-dans-eau ou eau-dans-huile classiques.

Le procédé conforme à la présente invention est un procédé de dispersion d'au moins une charge organique et/ou d'au moins une charge minérale pulvérulente dans un support se présentant sous la forme d'une dispersion huile-dans-eau ou eau-dans-huile constitué d'une phase grasse et d'une phase aqueuse
caractérisé par le fait qu'il comprend au moins une étape de mélange de la ou des charges dans ledit support au moyen d'un dispositif d'homogénéisation à haute pression en un ou plusieurs passages.

Les dispositifs d'homogénéisation à haute pression utilisés selon l'invention sont choisis parmi notamment ceux du type RANNIE®, GAULIN®, ou SOAVIE®.

Les pressions utilisées varient de préférence de 100 à 800 bars (de 1 à 8.10⁷ Pa) et plus particulièrement de 200 à 500 bars (de 2 à 5.10⁷ Pa).

La température d'homogénéisation utilisée varie de préférence de 20 à 85°C et pus particulièrement de 25 à 70°C.

Le support peut être constitué d'une seule phase grasse dans laquelle les charges pulvérulentes sont dispersées. On choisira de préférence une phase grasse constituée en général par un ou plusieurs composés choisis, seuls ou en mélanges, parmi les différents corps gras, les huiles d'origine végétale, animale ou minérale, les cires naturelles ou synthétiques, et analogues, cosmétiquement ou dermatologiquement acceptables.

Les supports particulièrement recherchés dans le cadre du procédé de l'invention sont des dispersions huile-dans-eau ou eau-dans-huile constituées d'une phase grasse et d'une phase aqueuse comme celles indiquées ci-dessus.

Dans le cadre des dispersions huile-dans-eau ou eau-dans-huile, une variante du procédé conforme à l'invention consiste à prédisperser la ou les charges dans une partie de la phase grasse de la dispersion huile-dans-eau ou eau-dans-huile à l'aide de l'homogénéisateur haute pression puis à remélanger le mélange ainsi obtenu à la quantité totale de la phase grasse avant le mélange final avec la phase aqueuse.

Dans le cadre des dispersions huile-dans-eau ou eau-dans-huile, une deuxième variante du procédé conforme à l'invention consiste à prédisperser la ou les charges dans la phase grasse de la dispersion huile-dans-eau ou eau-dans-huile à l'aide de l'homogénéisateur haute pression avant le mélange final avec la phase aqueuse.

Dans le cadre des dispersions huile-dans-eau ou eau-dans-huile, une troisième variante du procédé conforme à l'invention consiste à mélanger la ou les charges dans la dispersion huile-dans-eau ou eau-dans-huile préalablement réalisée à l'aide de l'homogénéisateur haute pression.

Selon chacune de ces trois variantes, l'étape de mélange final dans la phase aqueuse ou l'étape de réalisation préalable de la dispersion huile-dans-eau ou eau-dans-huile peuvent être effectuées à l'aide de l'homogénéisateur haute pression ou de moyens classiques d'agitation tels qu'un appareil du type Moritz.

Un autre objet de l'invention consiste en une composition cosmétique ou dermatologique sous forme de dispersion d'au moins une charge minérale pulvérulente dans un support constitué d'au moins une phase grasse, caractérisée par le fait qu'elle est susceptible d'être obtenue selon le procédé de dispersion tel que décrit ci-dessus.

Les compositions cosmétiques ou dermatologiques selon l'invention sont plus préférentiellement des dispersions eau-dans-huile ou huile-dans-eau et plus préférentiellement des émulsions eau-dans-huile ou huile-dans-eau. Les dernières sont plus particulièrement utilisées. En effet, pour des raisons cosmétiques et de confort d'utilisation, les émulsions huile-dans-eau sont les plus demandées dans le domaine de la cosmétique du fait qu'elles apportent sur la peau à l'application un toucher plus doux, moins gras et plus léger que les systèmes de dispersions eau-dans-huile.

Les charges utilisées dans les compositions de l'invention sont des particules cosmétiques ou dermatologiques insolubles dans le milieu de la composition.

Les charges utilisées selon l'invention peuvent être choisies parmi les charges minérales ou organiques, de structure lamellaire ou sphérique ou leurs mélanges. Elles peuvent être compactables ou difficilement compactables. Par charge difficilement compactable, on entend une matière première qui, à partir d'un certain pourcentage qui dépendra de la matière en question, ne peut être compactée au moyen d'une presse mécanique.

Chaque type de charges permet d'apporter des qualités particulières et différentes à la composition selon l'invention. Ainsi, par exemple, les charges de type lamellaires minérales apportent généralement de la douceur, les charges de type sphériques minérales apportent généralement un bon délitage et les charges sphériques organiques ont généralement un rôle structurant et apportent de la douceur.

Parmi les charges de type minérales lamellaires, on peut citer :
- les talcs ou silicates de magnésium hydratés, sous forme de particules de dimensions généralement inférieures à 40 µm ;
- les micas ou aluminosilicates de compositions variées et qui se présentent sous forme d'écailles ayant des dimensions de 2 à 200 µm, de préférence 5-70 µm et une épaisseur de 0,1 à 5 µm, de préférence de 0,2-3 µm, ces micas pouvant être d'origine naturelle (par exemple muscovite, margarite, roscoelite, lépidolite, biotite) ou d'origine synthétique. Ils sont généralement transparents et permettent de conférer à la peau un aspect satiné ;
- les argiles telles que les séricites, qui appartiennent à la même classe chimique et cristalline que la muscovite mais dont les propriétés organoleptiques sont proches du talc ;
- le kaolin ou silicate d'aluminium hydraté, qui se présente sous la forme de particules de formes isotropes ayant des dimensions généralement inférieures à 30µm et qui possèdent de bonnes propriétés d'absorption des corps gras ;
- les nitrures de bore.

Ces charges sont généralement compactables.

Toutefois, parmi ces charges de type lamellaires minérales, certaines sont difficilement compactables. On peut ainsi citer :
- certains talcs, tels que le 'Talc K1' de la société NIPPON ou le 'Talc Extra Steamic OOS' de la société LUZENAC ;
- certaines séricites, telles que la 'Séricite BC282' de la société WHITTAKER ;
- la plupart des micatitanes lorsqu'on les utilise à un fort pourcentage, parmi lesquels on peut citer le mica-nanotitane 'Coverleaf PC 2055M' de la société IKEDA.

Parmi les charges de type lamellaires organiques compactables, on peut citer les poudres de polymères de tétrafluoroéthylène, telles que le 'Fluon' de la société MONTEFLUOS, ou le 'Hostaflonq' de la société HOECHST.

Parmi les charges de type lamellaires organiques difficilement compactables, on peut citer la lauroyl-lysine 'Aminhope LL-11' de la société AJINOMOTO.

Parmi les charges de type sphériques minérales compactables, on peut citer :
- les oxydes de zinc et de titane, généralement utilisés sous la forme de particules ayant des dimensions ne dépassant pas quelques micromètres (ou même inférieures à 1 µm dans le cas de l'oxyde de titane), en particulier des dioxydes de titane sphériques comme le 'SPHERITITAN' de la société IKEDA ; ces oxydes ont un toucher onctueux, un bon pouvoir couvrant et une opacité importante ;
- le carbonate de calcium précipité qui, sous forme de particules de dimensions supérieures à 10 µm, a un toucher onctueux et permet d'obtenir un aspect mat ;
- le carbonate et l'hydrogénocarbonate de magnésium, qui possèdent, notamment, des propriétés de fixation des parfums ;
- la silice sphérique non poreuse et
- l'hydroxyapatite.

Parmi les charges de type minérales sphériques difficilement compactables, on peut citer :
- les microsphères de silice à porosité ouverte ou, de préférence, les microsphères de silice creuses, telles que les 'SILICA BEADS' de la société MAPRECOS, ces microsphères étant avantageusement imprégnées d'un actif cosmétique, et
- les microcapsules de verre ou de céramique 'MACROLITE' de la société 3M ;
   Parmi les charges de type organiques sphériques compactables, on peut citer :
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm, ont un toucher onctueux et facilitent l'adhérence de la poudre à la peau ;
- les poudres de polymères synthétiques non expansés, tels que le polyéthylène, les polyesters (par exemple isophtalate ou téréphtalate de polyéthylène) et les polyamides (par exemple le Nylon), sous la forme de particules ayant des dimensions inférieures à 50 µm, qui possèdent des propriétés absorbantes et permettent de conférer à la peau un aspect velouté ;
- les poudres de polymères synthétiques, réticulés ou non, sphéronisées comme les poudres d'acide polyacrylique ou polyméthacrylique, les poudres de polystyrène réticulé par le divinylbenzène et les poudres de résine de silicone, et
- des poudres de matériaux organiques d'origine naturelle comme l'octenylsuccinate d'amidon vendu sous le nom DRY FLOW PLUS par la société AMYLUM.

Parmi les charges de type organiques sphériques difficilement compactables, on peut citer :
- les microsphères microporeuses de polymères, qui ont une structure analogue à celle d'une éponge ; elles ont, en général, une surface spécifique d'au moins 0,5 m²/g et, en particulier, d'au moins 1 m²/g, ladite surface spécifique n'ayant pas de limite supérieure autre que celle résultant de la possibilité pratique de réaliser des microsphères de porosité très élevée : la surface spécifique peut, par exemple, atteindre 1 000 m²/g ou même davantage. On peut citer les microsphères de polymères acryliques, telles que celles en copolymère d'acrylate réticulé 'Polytrap' de la société DOW CORNING, et celles de polyméthacrylate de méthyle 'MICROPEARL M' ou 'MICROPEARL M 100' de la société SEPPIC ; ces microsphères microporeuses peuvent avantageusement être imprégnées, notamment par des actifs cosmétiques : on peut citer, à cet égard, les microsphères de copolymères de styrène-divinylbenzène vendues sous la dénomination commerciale 'PLASTIC POWDER FPSQ' par la société TOSHIKI, qui sont imprégnées de squalane qui est un actif cosmétique émollient ;
- les microcapsules de polymères ; qui comportent une seule cavité fermée et forment un réservoir, qui peut contenir un liquide, notamment un actif cosmétique ; elles sont préparées par des procédés connus tels que ceux décrits dans le brevet US-A 3 615 972 et EP-A 0 56219. Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène ; à titre d'exemple, on peut citer les microcapsules faites de polymères ou copolymères d'acrylate ou de méthacrylate de méthyle, ou encore de copolymères de chlorure de vinylidène et d'acrylonitrile ; parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique ; on peut également utiliser des polymères ou copolymères acryliques réticulés, par exemple dans le cas de polymères comportant un groupement carboxylique, par des diols servant d'agents réticulants; à titre d'exemple, on peut citer les microcapsules en copolymère de chlorure de vinylidène-acrylonitrile 'EXPANCEL' de la société Casco Nobel, les microcapsules 'Q-MAX' de la société Q-MAX et les microcapsules '3 M' de la société 3M.

Les charges dispersées selon le procédé conforme à l'invention peuvent être des pigments organiques, des pigments minéraux, enrobés ou non enrobés ou des pigments nacrés ou leurs mélanges.

Parmi les pigments organiques, on peut citer les laques telles que les laques de calcium de D et C Red n° 7, les laques de baryum des D et C Red n° 6 et 9, les laques d'aluminium des D et C Red n° 3 et de D et C Yellow n° 5 et les laques de zirconium de D et C Orange n° 5.

Parmi les pigments minéraux, on peut citer les oxydes de fer (rouge, brun, noir et jaune), les oxydes de chrome, les outre-mers (polysulfures d'aluminosilicates), le pyrophosphate de manganèse et le bleu de prusse (ferrocyanure ferrique), les oxydes de titane.

Les pigments nacrés peuvent être choisis, notamment, parmi les pigments nacrés blancs tels que le mica recouvert d'oxyde de titane ou l'oxychlorure de bismuth; les pigments nacrés colorés tels que le micatitane avec des oxydes de fer, le micatitane avec du bleu ferrique ou de l'oxyde de chrome, le micatitane avec un pigment organique du type précité, ainsi que les pigments à base d'oxychlorure de bismuth.

Les charges utilisées selon l'invention sont plus particulièrement choisies parmi les nanopigments minéraux à base d'oxydes métalliques, enrobés ou non enrobés.

Les oxydes métalliques utilisables dans le cadre de la présente invention peuvent être notamment choisis parmi les oxydes de titane, de zinc, de fer, de zirconium, de cérium ou leurs mélanges. La taille moyenne des particules primaires de ces oxydes varie de préférence de 5 à 100 nm et plus préférentiellement de 10 à 50 nm.

De tels nanopigments d'oxydes métalliques, enrobés ou non enrobés, sont des produits déja bien connus de l'homme de l'art et sont en particulier décrits dans la demande de brevet EP-A- 0 518 773, dont l'enseignement est ici inclus à titre de référence. A titre de nanopigments commerciaux utilisables dans le cadre de la présente invention, on peut mentionner les produits vendus sous les noms de marque UV-TITAN M 160, UV-TITAN M 212 et UV-TITAN M 262 par la Société KEMIRA et MICROTITANIUM DIOXIDE-MT-100T par la Société TAYCA.

Selon un mode préféré de réalisation des compositions cosmétiques ou dermatologiques selon l'invention, on fait appel à des nanopigments minéraux à base d'oxyde de titane, qui offrent en effet la meilleure efficacité au niveau photoprotection. Cet oxyde de titane peut se présenter sous une forme cristallisée de type rutile et/ou anatase, et/ou sous une forme amorphe ou substantiellement amorphe. Comme indiqué précédemment, ce pigment peut être alors enrobé ou non enrobé, mais de préférence on utilise des pigments enrobés, par exemple par de l'alumine et/ou du stéarate d'aluminium.

Les charges sont présentes dans les compositions de l'invention dans des concentrations allant préférentiellement de 0,1 à 70% en poids selon l'application envisagée.

La nature de la phase grasse rentrant dans la composition selon l'invention n'est pas critique et elle peut ainsi être constituée par tous les composés qui sont déja connus de façon générale comme convenant pour la fabrication de dispersions cosmétiques de type huile-dans-eau ou de type eau-dans-huile. En particulier, ces composés peuvent être choisis, seuls ou en mélanges, parmi les différents corps gras, les huiles d'origine végétale, animale ou minérale, les cires naturelles ou synthétiques, et analogues.

Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-α-oléfines.

Comme autres huiles utilisables dans les émulsions selon l'invention, on peut encore citer les benzoates d'alcools gras en C₁₂-C₁₅ (Finsolv TN de FINETEX), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, notamment ceux qui sont déja utilisés de manière habituelle dans la fabrication et l'obtention des compositions.

De manière classique, la phase aqueuse peut être constituée par de l'eau, ou un mélange d'eau et d'alcool(s) polyhydrique(s) comme par exemple glycérol, propylèneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvants cosmétiques classiques hydrosolubles.

Parmi les adjuvants cosmétiques classiques susceptibles d'être contenus dans la phase aqueuse et/ou dans la phase grasse des dispersions conformes à l'invention (selon leur caractère hydro- et/ou liposoluble), on peut citer notamment les épaississants ioniques ou non ioniques, des filtres solaires organiques classiques actifs dans l'UV-A et/ou l'UV-B hydrophiles ou lipophiles, les adoucissants, les antioxydants, les opacifiants, les stabilisants, les émollients, les agents hydratants, les vitamines, les parfums, les conservateurs, les séquestrants, les colorants, des plastifiants ou tout autre ingrédient habituellement utilisé dans le domaine cosmétique.

Les compositions conformes à l'invention contiennent de préférence en plus des tensio-actifs émulsionnants pour la préparation et l'obtention de la dispersion. Elles peuvent en outre contenir des co-émulsionnants dont le rôle est, lors de la préparation de la dispersion, de diminuer de manière substantielle la quantité d'agents tensio-actifs émulsionnants utilisée pour la réalisation de la dispersion.

Les systèmes émulsionnants convenant à la présente invention peuvent être des émulsionnants de type non ioniques et, plus particulièrement encore, être choisis parmi les alcools gras polyoxyéthylénés et/ou polyoxypropylénés (i.e des composés obtenus par réaction entre un alcool gras aliphatique, comme l'alcool béhénique ou l'alcool cétylique, et de l'oxyde d'éthylène ou de l'oxyde de propylène ou un mélange oxyde d'éthylène/oxyde de propylène) et les esters d'acides gras et de polyols, éventuellement polyoxyéthylénés et/ou polyoxypropylénés (i.e des composés obtenus par réaction d'un acide gras, comme l'acide stéarique ou l'acide oléique, avec un polyol, comme par exemple un alkylèneglycol ou du glycérol ou un polyglycérol, éventuellement en présence d'oxyde d'éthylène ou d'oxyde de propylène ou d'un mélange oxyde d'éthylène/oxyde de propylène), ou leurs mélanges. Ils sont présents dans des concentrations allant préférentiellement de 0,5 à 40 % en poids, de préférence de 2 à 15% en poids, par rapport à poids total de la composition.

A titre indicatif,
- les compositions conformes à l'invention sous forme d'émulsion eau-dans-huile présentent préférentiellement les formulations suivantes :
   (i) phase aqueuse : de 5 à 70 % en poids, plus particulièrement de 10 à 50% en poids, par rapport à l'ensemble de la formulation,
   (ii) phase huileuse : de 30 à 95 % en poids, plus particulièrement de 40 à 85% en poids, par rapport à l'ensemble de la formulation,
- les compositions conformes à l'invention sous forme d'émulsion huile-dans-eau présentent préférentiellement les formulations suivantes :
   (i) phase aqueuse : de 5 à 95 % en poids, plus particulièrement de 10 à 85% en poids, par rapport à l'ensemble de la formulation,
   (ii) phase huileuse : de 1 à 50 % en poids, plus particulièrement de 5 à 30% en poids, par rapport à l'ensemble de la formulation.

Un autre objet de l'invention consiste en l'utilisation des compositions telles que définies précédemment comme base de produit pour le soin et/ou le maquillage et/ou l'hygiène.

Parmi les compositions de soin, on peut citer les crèmes, les laits, les lotions, les gels de soin pour le corps, le visage, les mains ou les lèvres.

Parmi les compositions d'hygiène, on peut citer les démaquillants, les produits de rasage, les déodorants, les crèmes, les laits ou les gels pour le bain ou la douche.

Parmi les compositions de maquillage, on peut citer les fonds de teint, les crèmes teintées, les correcteurs, les embellisseurs de teint, les mascaras, les eye-liner, les rouge-à lèvres.

Un autre objet de l'invention consiste en l'utilisation des compositions telles que définies précédemment comme base de produit pour la protection de la peau et/ou des cheveux et/ou du cuir chevelu et/ou des ongles et/ou des cils et/ou des sourcils et/ou les muqueuses contre les effets des rayons ultraviolets en particulier les crèmes, les laits ou les gels antisolaires.

Les produits obtenus à partir des compositions de l'invention peuvent se présenter sous forme d'émulsion huile-dans-eau ou eau-dans-huile plus ou moins épaissie, de gel, de crème, de lait, de sérum, de lotion, de produit sous forme solide ou pâteuse.

L'invention porte également sur un procédé de traitement cosmétique de la peau et/ou des cheveux et/ou du cuir chevelu et/ou des ongles et/ou des cils et/ou des sourcils et/ou des muqueuses, caractérisée par le fait qu'on applique sur lesdits supports, une composition telle que définie précédemment.

L'invention porte également sur un procédé de traitement cosmétique pour la protection de la peau et/ou du cuir chevelu et/ou des ongles et/ou des cils et/ou des sourcils et/ou des muqueuses contre les effets des radiations ultraviolets, caractérisé par le fait qu'on applique sur lesdits supports, une composition telle que définie précédemment.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.
Les exemples ci-après, donnés à titre purement illustratif et non limitatif permettront de mieux comprendre l'invention.

### EXEMPLES 1 à 7 : Emulsions huile-dans-eau

Les exemples 1 à 7 qui suivent sont des émulsions huile-dans-eau obtenues selon le mode opératoire suivant:

On incorpore les nanopigments minéraux dans la phase grasse. On chauffe le mélange à 60-70°C et on homogénéise le tout à l'aide d'un homogénéisateur haute pression (pression 500 bars) du type RANNIE en un passage et plus si nécessaire. On rajoute au mélange homogène ainsi obtenu les tensio-actifs émulsionnants. On chauffe parallèlement la phase aqueuse à la même température puis on incorpore le mélange phase grasse/nanopigments/tensio-actifs dans la phase aqueuse et on réalise l'émulsion par une agitation par cisaillement avec un appareil du type Moritz.

### EXEMPLE 1: Crème de soin pour la peau

| *Phase grasse* | |
|---|---|
| - Huile de silicone volatile | 10,0 g |
| - Huile de vaseline | 5,0 g |
| - Perhydrosqualène | 18,0 g |
| - Tensio-actifs émulsionnants | 8 g |
| - Alcool cétylique | 1,2 g |
| - Acide stéarique | 0,1 g |
| - Lanoline liquide | 4,0 g |
| - Nanoparticules d'oxyde de Titane | 1,0 g |

| *Phase aqueuse* | |
|---|---|
| - Conservateur | 0,3 g |
| - Antioxydants | 0,1 g |
| - Triéthanolamine | 0,3 g |
| - Eau déminéralisée qsp | 100 g |

### EXEMPLE 2 : Crème de soin pour la peau

| *Phase grasse* | |
|---|---|
| - Myristate d'isopropyle | 1,0 g |
| - Huile de germes de maïs | 2,0 g |
| - Tensio-actifs émulsionnants | 6,0 g |
| - Alcool cétylique | 3,0 g |
| - Acide stéarique | 3,0 g |
| - Nanoparticules d'oxyde de Titane | 0,5 g |

| *Phase aqueuse* | |
|---|---|
| - Epaississant | 0,2 g |
| - Conservateur | 0,3 g |
| - Parfum | 0,4 g |
| - Eau déminéralisée qsp | 100 g |

### EXEMPLE 3 : Gel émulsionné de soin pour la peau

| *Phase grasse* | |
|---|---|
| - Huile de silicone volatile | 3,0 g |
| - Huile de Purcellin | 7,0 g |
| - Tensio-actifs émulsionnants | 0,3 g |
| - Nanoparticules d'oxyde de Zinc | 0,5 g |

| *Phase aqueuse* | |
|---|---|
| - Epaississant | 0,6 g |
| - Conservateur | 0,3 g |
| - Parfum | 0,4 g |
| - Alcool éthylique | 10 g |
| - Triéthanolamine | 0,2 g |
| - Eau déminéralisée qsp | 100 g |

### EXEMPLE 4 : Crème de soin pour la peau

| *Phase grasse* | |
|---|---|
| - Huile de silicone volatile | 5,0 g |
| - Huile de vaseline | 10,0 g |
| - Alcool de Lanoline | 1,5 g |
| - Tensio-actifs émulsionnants | 3,0 g |
| - Alcool cétylique | 0,5 g |
| - Acide stéarique | 0,72 g |
| - Nanoparticules d'oxyde de Fer Rouge | 0,5 g |

| *Phase aqueuse* | |
|---|---|
| - Epaississant | 0,3 g |
| - Conservateur | 0,16 g |
| - Triéthanolamine | 0,45 g |
| - Eau déminéralisée qsp | 100 g |

### EXEMPLE 5 : Crème de soin pour la peau

| *Phase grasse* | |
|---|---|
| - Huile de silicone volatile | 10,0 g |
| - Huile de vaseline | 5,0 g |
| - Alcool de Lanoline | 1,5 g |
| - Tensio-actifs émulsionnants | 3,0 g |
| - Alcool cétylique | 0,5 g |
| - Acide stéarique | 0,72 g |
| - Nanoparticules d'oxyde de Fer Jaune | 0,5 g |

| *Phase aqueuse* | |
|---|---|
| - Epaississant | 0,3 g |
| - Conservateur | 1,16 g |
| - NaOH | 0,45 g |
| - Glycérine | 7,0 g |
| - Eau déminéralisée qsp | 100 g |

### EXEMPLE 6 : Crème de soin pour la peau

| *Phase grasse* | |
|---|---|
| - Myristate d'isopropyle | 10,0 g |
| - Huile de silicone | 9,0 g |
| - Tensio-actif émulsionnant | 5,0 g |
| - Alcool cétylique | 9,0 g |
| - Acide stéarique | 1,0 g |
| - Nanoparticules d'oxyde de Titane | 5,0 g |

| *Phase aqueuse* | |
|---|---|
| - Epaississant | 0,5 g |
| - Conservateur | 0,3 g |
| - Eau déminéralisée qsp | 100g |

### EXEMPLE 7 : Crème de soin pour la peau

| *Phase grasse* | |
|---|---|
| - Huile de silicone volatile | 10,0 g |
| - Huile de vaseline | 10,0 g |
| - Huile de germes de maïs | 4,0 g |
| - Tensio-actif émulsionnant | 2,3 g |
| - Nanoparticules d'oxyde de Titane | 1,5 g |

| *Phase aqueuse* | |
|---|---|
| - Epaississant | 0,4 g |
| - Conservateur | 0,3 g |
| - Triéthanolamine | 0,4 g |
| - Eau déminéralisée qsp | 100 g |

### EXEMPLES 8 ET 9 : Emulsions eau-dans-huile

Les exemples 8 et 9 qui suivent sont obtenus selon le mode opératoire suivant :

On incorpore les nanopigments minéraux dans la phase aqueuse. On chauffe le mélange à 60-70°C et on homogénéise le tout à l'aide d'un homogénéisateur haute pression (pression 500 bars) du type RANNIE en un passage et plus si nécessaire. On chauffe à la même température parallèlement la phase grasse contenant les tensio-actifs émulsionnants puis on incorpore le mélange phase grasse avec la phase aqueuse et on réalise l'émulsion par une agitation par cisaillement avec un appareil du type Moritz.

### EXEMPLE 8 : Crème de soin pour la peau

| *Phase grasse* | |
|---|---|
| - Huile de tournesol | 15,0 g |
| - Huile de vaseline | 10,0 g |
| - Composition arômatique | 1,0 g |
| - Tensio-actif émulsionnant | 20 g |

| *Phase aqueuse* | |
|---|---|
| - Conservateur | 0,3 g |
| - Glycérol | 5,0 g |
| - Sulfate de magnésium | 0,5 g |
| - Nanoparticules d'oxyde de Titane | 5,0 g |
| - Eau déminéralisée qsp | 100 g |

### EXEMPLE 9 : Crème de soin pour la peau

| *Phase grasse* | |
|---|---|
| - Huile de silicone volatile | 8,0 g |
| - Huile de vaseline | 5,0 g |
| - Huile de purcellin | 14,0 g |
| - Myristate d'isopropyle | 5,0 g |
| - Tensio-actif émulsionnant | 5,4 g |

| *Phase aqueuse* | |
|---|---|
| - Chlorure de sodium | 0,5 g |
| - Nanoparticules d'oxyde de Titane | 5,0 g |
| - Conservateur | 0,3 g |
| - Eau déminéralisée qsp | 100 g |

### EXEMPLES COMPARATIFS

On mesure le diamètre moyen des amas de nano-oxyde de titane (charges) formés au cours de la dispersion de ces pigments dans une émulsion eau-dans-huile et dans une monophase huileuse.

On compare dans un premier temps une émulsion eau-dans-huile **A** à base desdits pigments obtenue selon le procédé de l'invention au moyen d'un homogénéisateur haute pression avec une émulsion eau-dans-huile **B** à base desdits pigments obtenue selon un procédé classique d'homogénéisation.

On compare dans un deuxième temps une monophase huileuse **C** à base desdits pigments obtenue selon le procédé de l'invention utilisant un homogénéisateur haute pression avec une monophase huileuse **D** à base desdits pigments obtenue selon un procédé classique d'homogénéisation.

On mesure pour chaque composition **A**, **B**, **C**, **D** le diamètre moyen des amas de pigment (exprimé en nanomètres) au moyen d'un granulomètre du type BROOKHAVEN INSTRUMENTATION de référence Bl 90 PARTICULE SIZER.

Plus la taille des diamètres mesurée est petite, plus la dispersion des pigments est fine et homogène.

### Formulation générale des émulsions A et B

| *Phase 1* | |
|---|---|
| - Glycérine | 3 g |
| - Eau déminéralisée | qs |

| *Phase 2* | |
|---|---|
| - Tensio-actif émulsionnant | 5 g |
| - Alcool stéarylique | 1 g |
| - Alcool cétylique | 0,5 g |
| - Octanoate de cétéaryle | 4 g |
| - Fraction liquide de beurre de karité | 6 g |
| - Huile de silicone | 9 g |
| - Conservateur | 0,2 g |
| - Nano-oxyde de titane | 5 g |

| *Phase 3* | |
|---|---|
| - Conservateur | 0,3 g |
| - Epaississant | 1,2 g |
| - Eau déminéralisée qsp | 100 g |

### Mode opératoire des émulsions A et B

| **Mode opératoire de l'émulsion A selon l'invention** | **Mode opératoire de l'émulsion B selon l'art antérieur** |
|---|---|
| On chauffe la phase 1 à 70°C. | idem |
| On chauffe la phase 2 à 65°C et on laisse sous agitation classique type RAYNERI pour mouiller le nano-oxyde de titane. | idem |
| On verse la phase 1 dans la phase 2 sous agitation classique type RAYNERI. | idem |
| On effectue un passage du mélange obtenu à l'homogénéisateur haute pression RANNIE à 65°C à 200 bars. | On homogénéise le mélange obtenu sous agitation par cisaillement du type MORITZ. |
| On laisse refroidir à température ambiante, on ajoute la phase 3 sous agitation par cisaillement du type MORITZ. | idem |

### Formulation générale des huiles C et D

| *Phase 1* | |
|---|---|
| - Alcool stéarylique | 1 g |
| - Octanoate de cétéaryle | 24,5 g |
| - Conservateur | 0,2 g |
| - Nano-oxyde de titane | 5 g |

### Mode opératoire des huiles C et D

| **Mode opératoire de l'huile C selon l'invention** | **Mode opératoire de l'huile D selon l'art antérieur** |
|---|---|
| On chauffe la phase 1 à 65°C. | idem |
| On laisse sous agitation classique type RAYNERI pour mouiller le nano-oxyde de titane. | idem |
| On effectue un passage du mélange obtenu à l'homogénéisateur haute pression RANNIE à 65°C à 200 bars. | On homogénéise le mélange obtenu sous agitation par cisaillement du type MORITZ. |
| On laisse refroidir à température ambiante. | idem |

Les résultats granulométriques sont indiqués dans le tableau suivant:

| **Type de formulation** | **Dispersion avec homogénéisation haute pression** | **Dispersion classique sans homogénéisation haute pression** |
|---|---|---|
| | **A** | **B** |
| Emulsion huile-dans-eau | | |
| | 537 nm | 1532 nm |

| | **C** | **D** |
|---|---|---|
| Monophase huileuse | | |
| | 3841 nm | 4178 nm |

On constate que les particules de nanotitane sont mieux dispersées dans les formulations A et C obtenues selon le procédé de l'invention utilisant un homogénéisateur haute pression que dans les formulations B et D obtenues selon un procédé classique.

## Revendications

1. Procédé de dispersion d'au moins une charge organique et/ou d'au moins une charge minérale pulvérulente dans un support se présentant sous la forme d'une dispersion huile-dans-eau ou eau-dans-huile constituée d'une phase grasse et d'une phase aqueuse, caractérisé par le fait qu'il comprend au moins une étape de mélange de la ou des charges dans ledit support au moyen d'un dispositif d'homogénéisation à haute pression en un ou plusieurs passages.

2. Procédé selon la revendication 1, caractérisé par le fait que la pression varie de 100 à 800 bars (de 1 à 8.10⁷ Pa) et plus particulièrement de 200 à 500 bars (de 2 à 5.10⁷ Pa).

3. Procédé selon l'une des revendications précédentes, caractérisé par le fait que la température d'homogénéisation varie de préférence de 20 à 85°C et pus particulièrement de 25 à 70°C.

4. Procédé selon l'une des revendications précédentes, caractérisé par le fait que la phase grasse est constituée par un ou plusieurs composés choisis, seuls ou en mélanges, parmi les différents corps gras, les huiles d'origine végétale, animale ou minérale, les cires naturelles ou synthétiques, et analogues, cosmétiquement ou dermatologiquement acceptables.

5. Procédé selon l'une des revendications précédentes, caractérisé par le fait qu'il consiste à prédisperser la ou les charges dans une partie de la phase grasse d'une dispersion huile-dans-eau ou eau-dans-huile à l'aide de l'homogénéisateur haute pression puis à remélanger le mélange ainsi obtenu à la quantité totale de la phase grasse avant d'effectuer le mélange final avec la phase aqueuse.

6. Procédé selon l'une des revendications précédentes, caractérisé par le fait qu'il consiste à prédisperser la ou les charges dans la phase grasse d'une dispersion huile-dans-eau ou eau-dans-huile à l'aide de l'homogénéisateur haute pression avant d'effectuer le mélange final avec la phase aqueuse.

7. Procédé selon l'une des revendications précédentes, caractérisé par le fait qu'il consiste à mélanger à l'aide de l'homogénéisateur haute pression la ou les charges dans la dispersion huile-dans-eau ou eau-dans-huile préalablement réalisée.

8. Procédé selon l'une des revendications précédentes, caractérisée par le fait que la ou les charges sont choisies parmi les charges minérales ou organiques, de structure lamellaire ou sphérique, compactables ou difficilement compactables et leurs mélanges.

9. Procédé selon la revendication 8, dans lequel les charges de type minérales lamellaires sont choisies parmi les talcs ou silicates de magnésium hydratés, les micas ou aluminosilicates, les argiles telles que les séricites, le kaolin ou silicate d'aluminium hydraté, les nitrures de bore, les micatitanes.

10. Procédé selon la revendication 8, dans lequel les charges de type lamellaires organiques sont des poudres de polymères de tétrafluoroéthylène ou la lauroyl-lysine.

11. Procédé selon la revendication 8, dans lequel les charges de type sphériques minérales sont choisies parmi les oxydes de zinc et de titane, le carbonate de calcium précipité, le carbonate et l'hydrocarbonate de magnésium, la silice sphérique non poreuse, l'hydroxyapatite, les microsphères de silice à porosité ouverte ou creuses, éventuellement imprégnées d'un actif cosmétique et les microcapsules de verre ou de céramique.

12. Procédé selon la revendication 8, dans lequel les charges de type organiques sphériques sont choisies parmi les savons métalliques dérivés d'acides organiques carboxyliques ayant de 8 à 22 atomes de carbone, tels que le stéarate de zinc, de magnésium ou de lithium, le laurate de zinc, le myristate de magnésium ; les poudres de polymères synthétiques non expansés; les poudres de polymères synthétiques réticulés ou non, sphéronisées ; les poudres de matériaux organiques d'origine naturelle ; les microsphères microporeuses de polymères, éventuellement imprégnées par des actifs cosmétiques; les microcapsules de polymères éventuellement réticulés.

13. Procédé selon l'une des revendications 1 à 7, caractérisé par le fait que la ou les charges pulvérulentes sont choisies parmi les pigments organiques, les pigments minéraux, enrobés ou non enrobés, les pigments nacrés ou leurs mélanges.

14. Procédé selon la revendication 13, caractérisé par le fait que la ou les charges pulvérulentes sont choisies parmi les nanopigments à base d'oxydes métalliques, enrobés ou non enrobés.

15. Procédé selon la revendication 14, caractérisé par le fait que les nanopigments minéraux sont choisis dans le groupe constitué par les oxydes de titane, les oxydes de zinc, les oxydes de fer, les oxydes de zirconium, les oxydes de cérium ou leurs mélanges.

16. Procédé selon l'une des revendications 14 à 15, caractérisé par le fait que la taille moyenne des particules primaires des oxydes métalliques varie de 5 à 100 nm et plus préférentiellement de 10 à 50 nm.

17. Dispersion d'au moins une charge minérale pulvérulente dans un support constitué d'au moins une phase grasse, ladite dispersion étant susceptible d'être obtenue par un procédé de dispersion comprenant au moins une étape de mélange de la ou des charges dans ledit support au moyen d'un dispositif d'homogénéisation à haute pression en un ou plusieurs passages, ladite charge minérale étant choisie parmi les nanopigments minéraux à base d'oxydes métalliques.

18. Composition cosmétique ou dermatologique sous forme de dispersion d'au moins une charge minérale pulvérulente dans un support constitué d'au moins une phase grasse, caractérisée par le fait qu'elle est susceptible d'être obtenue par un procédé de dispersion comprenant au moins une étape de mélange de la ou des charges dans ledit support au moyen d'un dispositif d'homogénéisation à haute pression en un ou plusieurs passages, ladite charge minérale étant choisie parmi les nanopigments minéraux à base d'oxydes métalliques.

19. Composition selon la revendication 18, caractérisée par le fait qu'elle est une dispersion huile-dans-eau ou eau-dans-huile constituée d'une phase grasse et d'une phase aqueuse.

20. Composition selon l'une des revendications 18 à 19, caractérisée par le fait qu'elle est une émulsion huile-dans-eau ou une émulsion eau-dans-huile.

21. Composition selon l'une des revendications 18 à 20, caractérisée par le fait que les nanopigments minéraux sont choisis dans le groupe constitué par les oxydes de titane, les oxydes de zinc, les oxydes de fer, les oxydes de zirconium, les oxydes de cérium ou leurs mélanges.

22. Composition selon l'une des revendications 18 à 21, caractérisée par le fait que la taille moyenne des particules primaires des oxydes métalliques varie de 5 à 100 nm et préférentiellement de 10 à 50 nm.

23. Composition selon l'une des revendications 18 à 22, caractérisée par le fait que les nanopigments minéraux sont choisis dans le groupe constitué par les oxydes de titane, enrobés ou non enrobés.

24. Composition selon l'une des revendications 18 à 23, caractérisée par le fait que la dispersion comprend en outre au moins une autre charge, choisie parmi les charges minérales différentes des nanopigments d'oxydes métalliques et les charges organiques.

25. Composition selon la revendication 24, caractérisée par le fait que ladite autre charge est choisie parmi les talcs ou silicates de magnésium hydratés; les micas ou aluminosilicates; les argiles telles que les séricites, le kaolin ou silicate d'aluminium hydraté, les nitrures de bore, les micatitanes; les poudres de polymères de tétrafluoroéthylène; la lauroyl-lysine; le carbonate de calcium précipité, le carbonate et l'hydrocarbonate de magnésium, la silice sphérique non poreuse, l'hydroxyapatite, les microsphères de silice à porosité ouverte ou creuses, éventuellement imprégnées d'un actif cosmétique, les microcapsules de verre ou de céramique, les savons métalliques dérivés d'acides organiques carboxyliques ayant de 8 à 22 atomes de carbone, tels que le stéarate de zinc, de magnésium ou de lithium, le laurate de zinc, le myristate de magnésium ; les poudres de polymères synthétiques non expansés; les poudres de polymères synthétiques réticulés ou non, sphéronisées; les poudres de matériaux organiques d'origine naturelle, les microsphères microporeuses de polymères, éventuellement imprégnées par des actifs cosmétiques ; les microcapsules de polymères éventuellement réticulés; les pigments organiques, les pigments minéraux, les pigments nacrés, et leurs mélanges.

26. Composition selon l'une quelconque des revendications 18 à 25, caractérisée en ce qu'elle contient de 0,1 à 70% en poids de charges(s) par rapport au poids total de la composition.

27. Composition selon l'une quelconque des revendications 18 à 26, caractérisée en ce qu'elle contient en outre au moins un agent émulsionnant.

28. Composition selon la revendication 27, caractérisée en ce que la teneur en agent(s) émulsionnant(s) est comprise entre 0,5 et 40 % en poids, de préférence entre 2 et 15 % en poids, par rapport au poids total de la composition.

29. Composition selon l'une quelconque des revendications 18 à 28, caractérisée en ce qu'elle contient en outre au moins un adjuvant choisi parmi les épaississants ioniques ou non ioniques, les filtres solaires organiques classiques actifs dans l'UV-A et/ou l'UV-B hydrophiles ou lipophiles, les adoucissants, les antioxydants, les opacifiants, les stabilisants, les émollients, les agents hydratants, les vitamines, les parfums, les conservateurs, les séquestrants, les colorants, des plastifiants ou tout autre ingrédient habituellement utilisé dans le domaine cosmétique.

30. Composition selon l'une quelconque des revendications 20 à 29, caractérisée en ce qu'elle est une émulsion eau-dans-huile et présente les caractéristiques suivantes :
(i) phase aqueuse : de 5 à 70 % en poids, plus particulièrement de 10 à 50% en poids, par rapport à l'ensemble de la formulation,
(ii) phase huileuse : de 30 à 95 % en poids, plus particulièrement de 40 à 85% en poids, par rapport à l'ensemble de la formulation.

31. Composition selon l'une quelconque des revendications 20 à 29, caractérisée en ce qu'elle est une émulsion huile-dans-eau et présente les caractéristiques suivantes :
(i) phase aqueuse : de 5 à 95 % en poids, plus particulièrement de 10 à 85% en poids, par rapport à l'ensemble de la formulation,
(ii) phase huileuse : de 1 à 50 % en poids, plus particulièrement de 5 à 30% en poids, par rapport à l'ensemble de la formulation.

32. Utilisation de la composition selon l'une des revendications 18 à 31 comme base de produit pour le soin et/ou le maquillage et/ou l'hygiène.

33. Utilisation de la composition selon l'une des revendications 18 à 31 comme base de produit pour la protection de la peau et/ou des cheveux et/ou du cuir chevelu et/ou des ongles et/ou des cils et/ou des sourcils et/ou les muqueuses contre les effets des rayons ultraviolets.

34. Procédé de traitement cosmétique de la peau et/ou des cheveux et/ou du cuir chevelu et/ou des ongles et/ou des cils et/ou des sourcils et/ou des muqueuses, caractérisé par le fait qu'on applique sur lesdits supports, une composition telle que définie selon l'une quelconque des revendications 18 à 33.

35. Procédé de traitement cosmétique pour la protection de la peau et/ou des cheveux et/ou du cuir chevelu et/ou des ongles et/ou des cils et/ou des sourcils et/ou des muqueuses contre les effets des radiations ultraviolets, caractérisé par le fait qu'on applique sur lesdits supports, une composition telle que définie selon l'une quelconque des revendications 18 à 33.

## Claims

1. Process for the dispersion of at least one pulvervlent organic filler and/or of at least one pulverulent inorganic filler in a vehicle which is provided in the form of an oil-in-water or water-in-oil dispersion composed of a fatty phase and of an aqueous phase, characterized in that it comprises at least one stage of mixing the filler or fillers in the said vehicle by means of a high-pressure homogenization device in one or a number of passes.

2. Process according to Claim 1, characterized in that the pressure varies from 100 to 800 bar (from 1 to 8 × 10⁷ Pa) and more particularly from 200 to 500 bar (from 2 to 5 × 10⁷ Pa).

3. Process according to one of the preceding claims, characterized in that the homogenization temperature preferably varies from 20 to 85°C and more particularly from 25 to 70°C.

4. Process according to one of the preceding claims, characterized in that the fatty phase is composed of one or a number of compounds chosen, alone or as mixtures, from the various fatty substances, oils of vegetable, animal or mineral origin, natural or synthetic waxes, and the like, which are cosmetically or dermatologically acceptable.

5. Process according to one of the preceding claims, characterized in that it comprises the predispersion of the filler or fillers in a portion of the fatty phase of an oil-in-water or water-in-oil dispersion using the high-pressure homogenizer and then the remixing of the mixture thus obtained with the entire amount of the fatty phase before carrying out the final mixing with the aqueous phase.

6. Process according to one of the preceding claims, characterized in that it comprises the predispersion of the filler or fillers in the fatty phase of an oil-in-water or water-in-oil dispersion using the high-pressure homogenizer before carrying out the final mixing with the aqueous phase.

7. Process according to one of the preceding claims, characterized in that it comprises the mixing, using the high-pressure homogenizer, of the filler or fillers in the oil-in-water or water-in-oil dispersion produced beforehand.

8. Process according to one of the preceding claims, characterized in that the filler or fillers are chosen from inorganic or organic fillers of lamellar or spherical structure which are compactable or difficult to compact and their mixtures.

9. Process according to Claim 8, in which the fillers of lamellar inorganic type are chosen from talcs or hydrated magnesium silicates, micas or aluminosilicates, clays, such as sericites, kaolin or hydrated aluminium silicate, boron nitrides or titanium oxide-coated micas.

10. Process according to Claim 8, in which the fillers of lamellar organic type are tetrafluoroethylene polymer powders or lauroyllysine.

11. Process according to Claim 8, in which the fillers of spherical inorganic type are chosen from zinc and titanium oxides, precipitated calcium carbonate, magnesium carbonate and hydrogencarbonate, non-porous spherical silica, hydroxyapatite, silica microspheres with an open porosity or hollow silica microspheres, optionally impregnated with a cosmetic active principle, and glass or ceramic microcapsules.

12. Process according to Claim 8, in which the fillers of spherical organic type are chosen from metal soaps derived from organic carboxylic acids having from 8 to 22 carbon atoms, such as zinc, magnesium or lithium stearate, zinc laurate or magnesium myristate; powders of non-expanded synthetic polymers; spheronized powders of crosslinked or non-crosslinked synthetic polymers; powders of organic materials of natural origin; microporous polymer microspheres, optionally impregnated with cosmetic active principles; or optionally crosslinked polymer microcapsules.

13. Process according to one of Claims 1 to 7, characterized in that the pulverulent filler or fillers are chosen from organic pigments, inorganic pigments, which are coated or non-coated, nacreous pigments or their mixtures.

14. Process according to Claim 13, characterized in that the pulverulent filler or fillers are chosen from nanopigments based on metal oxides which are coated or non-coated.

15. Process according to Claim 14, characterized in that the inorganic nanopigments are chosen from the group composed of titanium oxides, zinc oxides, iron oxides, zirconium oxides, cerium oxides or their mixtures.

16. Process according to one of Claims 14 and 15, characterized in that the mean size of the primary particles of the metal oxides varies from 5 to 100 nm and more preferentially from 10 to 50 nm.

17. Dispersion of at least one pulverulent inorganic filler in a vehicle composed of at least one fatty phase, the said dispersion being capable of being obtained by a dispersion process comprising at least one stage of mixing the filler or fillers in the said vehicle by means of a high-pressure homogenization device in one or a number of passes, the said inorganic filler being chosen from inorganic nanopigments based on metal oxides.

18. Cosmetic or dermatological composition in the form of a dispersion of at least one pulverulent inorganic filler in a vehicle composed of at least one fatty phase, characterized in that it is capable of being obtained by a dispersion process comprising at least one stage of mixing the filler or fillers in the said vehicle by means of a high-pressure homogenization device in one or a number of passes, the said inorganic filler being chosen from inorganic nanopigments based on metal oxides.

19. Composition according to Claim 18, characterized in that it is an oil-in-water or water-in-oil dispersion composed of a fatty phase and of an aqueous phase.

20. Composition according to one of Claims 18 and 19, characterized in that it is an oil-in-water emulsion or a water-in-oil emulsion.

21. Composition according to one of Claims 18 to 20, characterized in that the inorganic nanopigments are chosen from the group composed of titanium oxides, zinc oxides, iron oxides, zirconium oxides, cerium oxides or their mixtures.

22. Composition according to one of Claims 18 to 21, characterized in that the mean size of the primary particles of the metal oxides varies from 5 to 100 nm and preferentially from 10 to 50 nm.

23. Composition according to one of Claims 18 to 22, characterized in that the inorganic nanopigments are chosen from the group composed of coated or non-coated titanium oxides.

24. Composition according to one of Claims 18 to 23, characterized in that the dispersion additionally comprises at least one other filler chosen from inorganic fillers other than metal oxide nanopigments and organic fillers.

25. Composition according to Claim 24, characterized in that the said other filler is chosen from talcs or hydrated magnesium silicates; micas or aluminosilicates; clays, such as sericites, kaolin or hydrated aluminium silicate, boron nitrides or titanium oxide-coated micas; tetrafluoroethylene polymer powders; lauroyllysine; precipitated calcium carbonate, magnesium carbonate and hydrogencarbonate, non-porous spherical silica, hydroxyapatite, silica microspheres with an open porosity or hollow silica microspheres, optionally impregnated with a cosmetic active principle, glass or ceramic microcapsules, metal soaps derived from organic carboxylic acids having from 8 to 22 carbon atoms, such as zinc, magnesium or lithium stearate, zinc laurate or magnesium myristate; powders of non-expanded synthetic polymers; spheronized powders of crosslinked or non-crosslinked synthetic polymers; powders of organic materials of natural origin; microporous polymer microspheres, optionally impregnated with cosmetic active principles; optionally crosslinked polymer microcapsules; organic pigments, inorganic pigments or nacreous pigments, and their mixtures.

26. Composition according to any one of Claims 18 to 25, characterized in that it contains from 0.1 to 70% by weight of filler(s) with respect to the total weight of the composition.

27. Composition according to any one of Claims 18 to 26, characterized in that it additionally contains at least one emulsifying agent.

28. Composition according to Claim 27, characterized in that the content of emulsifying agent(s) is between 0.5 and 40% by weight, preferably between 2 and 15% by weight, with respect to the total weight of the composition.

29. Composition according to any one of Claims 18 to 28, characterized in that it additionally contains at least one adjuvant chosen from ionic or non-ionic thickeners, conventional hydrophilic or lipophilic organic sunscreening agents which are active in the UV-A and/or the UV-B, softeners, antioxidants, opacifiers, stabilizers, emollients, moisturizing agents, vitamins, fragrances, preservatives, sequestering agents, dyes, plasticizers or any other ingredient commonly used in the cosmetics field.

30. Composition according to any one of Claims 20 to 29, characterized in that it is a water-in-oil emulsion and has the following characteristics:
(i) aqueous phase: from 5 to 70% by weight, more particularly from 10 to 50% by weight, with respect to the whole of the formulation,
(ii) oily phase: from 30 to 95% by weight, more particularly from 40 to 85% by weight, with respect to the whole of the formulation.

31. Composition according to any one of Claims 20 to 29, characterized in that it is an oil-in-water emulsion and has the following characteristics:
(i) aqueous phase: from 5 to 95% by weight, more particularly from 10 to 85% by weight, with respect to the whole of the formulation,
(ii) oily phase: from 1 to 50% by weight, more particularly from 5 to 30% by weight, with respect to the whole of the formulation.

32. Use of the composition according to one of Claims 18 to 31 as the basis for a care and/or make-up and/or hygiene product.

33. Use of the composition according to one of Claims 18 to 31 as the basis for a product for protecting the skin and/or hair and/or scalp and/or nails and/or eyelashes and/or eyebrows and/or mucous membranes against the effects of ultraviolet radiation.

34. Process for the cosmetic treatment of the skin and/or hair and/or scalp and/or nails and/or eyelashes and/or eyebrows and/or mucous membranes, characterized in that a composition as defined according to any one of Claims 18 to 33 is applied to the said substrates.

35. Cosmetic treatment process for protecting the skin and/or hair and/or scalp and/or nails and/or eyelashes and/or eyebrows and/or mucous membranes against the effects of ultraviolet radiation, characterized in that a composition as defined according to any one of Claims 18 to 33 is applied to the said substrates.

## Patentansprüche

1. Verfahren zum Dispergieren mindestens eines organischen Füllstoffs und/oder mindestens eines pulverförmigen anorganischen Füllstoffs in einem Träger, der in Form einer Öl-in-Wasser-Dispersion oder einer Wasser-in-Öl-Dispersion vorliegt, welche aus einer Fettphase und einer wässerigen Phase besteht, dadurch gekennzeichnet, daß es mindestens einen Schritt umfaßt, bei dem der oder die Füllstoffe mit einer Vorrichtung zum Hochdruckhomogenisieren in einem oder mehreren Durchgängen in den Träger gemischt werden.

2. Verfahren nach Anspruch 1, dadurch gekennzeichnet, daß der Druck im Bereich von 100 bis 800 bar (1 bis 8·10⁷ Pa) und insbesondere von 200 bis 500 bar (2 bis 5·10⁷ Pa) liegt.

3. Verfahren nach einem der vorhergehenden Ansprüche, dadurch gekennzeichnet, daß die Homogenisiertemperatur vorzugsweise im Bereich von 20 bis 85 °C und insbesondere von 25 bis 70 °C liegt.

4. Verfahren nach einem der vorhergehenden Ansprüche, dadurch gekennzeichnet, daß die Fettphase aus einer oder mehreren Verbindungen besteht, welche, allein oder im Gemisch, ausgewählt sind unter den verschiedenen Fettsubstanzen, Ölen pflanzlicher, tierischer oder mineralischer Herkunft, natürlichen oder synthetischen Wachsen und dergleichen, die kosmetisch oder dermatologisch akzeptabel sind.

5. Verfahren nach einem der vorhergehenden Ansprüche, dadurch gekennzeichnet, daß es darin besteht, den oder die Füllstoffe mit einem Hochdruckhomogenisator in einem Teil der Fettphase einer Öl-in-Wasser-Dispersion oder einer Wasser-in-Öl-Dispersion vorzudispergieren und dann das so erhaltene Gemisch mit der Gesamtmenge der Fettphase zu vermischen, wonach schlußendlich ein Gemisch mit der wässerigen Phase erzeugt wird.

6. Verfahren nach einem der vorhergehenden Ansprüche, dadurch gekennzeichnet, daß es darin besteht, den oder die Füllstoffe mit einem Hochdruckhomogenisator in der Fettphase einer Öl-in-Wasser-Dispersion oder einer Wasser-in-Öl-Dispersion vorzudispergieren, wonach mit der wässerigen Phase vermischt wird.

7. Verfahren nach einem der vorhergehenden Ansprüche, dadurch gekennzeichnet, daß es darin besteht, den oder die Füllstoffe mit einem Hochdruckhomogenisator in die zuvor hergestellte Öl-in-Wasser-Dispersion oder Wasser-in-Öl-Dispersion zu mischen.

8. Verfahren nach einem der vorhergehenden Ansprüche, dadurch gekennzeichnet, daß der oder die Füllstoffe unter verdichtbaren oder schwer verdichtbaren anorganischen oder organischen Füllstoffen mit lamellarer oder kugelförmiger Struktur und Gemischen dieser Füllstoffe ausgewählt sind.

9. Verfahren nach Anspruch 8, worin die Füllstoffe vom lamellaren anorganischen Typ unter den Talken oder hydratisierten Magnesiumsilicaten, Glimmern oder Aluminosilicaten, Tonen, wie den Sericiten, Kaolin oder hydratisiertem Aluminiumsilicat, Bornitriden und Titandioxidglimmern ausgewählt sind.

10. Verfahren nach Anspruch 8, worin die Füllstoffe vom organischen lamellaren Typ Pulver aus Tetrafluorethylenpolymeren oder Lauroyllysin sind.

11. Verfahren nach Anspruch 8, worin die Füllstoffe vom anorganischen kugelförmigen Typ ausgewählt sind unter den Oxiden von Zink und Titan, gefälltem Calciumcarbonat, Magnesiumcarbonat oder Magnesiumhydrogencarbonat, nichtporösem kugelförmigem Siliciumdioxid, Hydroxylapatit, offenporigen oder hohlen Mikrokugeln aus Siliciumdioxid, die gegebenenfalls mit einem kosmetischen Wirkstoff imprägniert sind, und Mikrokapseln aus Glas oder Keramik.

12. Verfahren nach Anspruch 8, worin die Füllstoffe vom kugelförmigen organischen Typ ausgewählt sind unter den Metallseifen, die von organischen Carbonsäuren mit 8 bis 22 Kohlenstoffatomen abgeleitet sind, wie Zinkstearat, Magnesiumstearat oder Lithiumstearat, Zinklaurat und Magnesiummyristat, Pulvern von nichtexpandierten synthetischen Polymeren, Pulvern von gegebenenfalls vernetzten kugelförmigen synthetischen Polymeren, Pulvern von organischen Materialien natürlicher Herkunft, mikroporösen Polymermikrokugeln, die gegebenenfalls mit kosmetischen Wirkstoffen imprägniert sind, und gegebenenfalls vernetzten Polymermikrokapseln.

13. Verfahren nach einem der Ansprüche 1 bis 7, dadurch gekennzeichnet, daß der oder die pulverförmigen Füllstoffe ausgewählt sind unter organischen Pigmenten, gegebenenfalls umhüllten anorganischen Pigmenten, Perlglanzpigmenten oder Gemischen dieser Pigmente.

14. Verfahren nach Anspruch 13, dadurch gekennzeichnet, daß der oder die pulverförmigen Füllstoffe unter gegebenenfalls umhüllten Nanopigmenten auf der Basis von Metalloxiden ausgewählt sind.

15. Verfahren nach Anspruch 14, dadurch gekennzeichnet, daß die anorganischen Nanopigmente ausgewählt sind unter den Titandioxiden, Zinkoxiden, Eisenoxiden, Zirconiumdioxiden und Cerdioxiden oder den Gemischen dieser Oxide.

16. Verfahren nach Anspruch 14 bis 15, dadurch gekennzeichnet, daß die mittlere Größe der Primärpartikel der Metalloxide im Bereich von 5 bis 100 nm und vorzugsweise von 10 bis 50 nm liegt.

17. Dispersion mindestens eines pulverförmigen anorganischen Füllstoffs in einem Träger, der mindestens aus einer Fettphase besteht, wobei diese Dispersion durch ein Dispergierverfahren erhalten werden kann, daß mindestens einen Schritt umfaßt, bei dem der oder Füllstoffe in einem oder mehreren Durchgängen mit einer Vorrichtung zum Hochdruckhomogenisieren in den Träger gemischt werden, wobei der anorganische Füllstoff unter den anorganischen Nanopigmenten auf der Basis von Metalloxiden ausgewählt ist.

18. Kosmetische oder dermatologische Zusammensetzung in Form einer Dispersion mindestens eines pulverförmigen anorganischen Füllstoffs in einem Träger, der mindestens aus einer Fettphase besteht, dadurch gekennzeichnet, daß sie durch ein Dispergierverfahren erhalten werden kann, das mindestens einen Schritt umfaßt, bei dem der oder die Füllstoffe in einem oder mehreren Durchgängen mit einer Vorrichtung zum Hochdruckhomogenisieren in den Träger gemischt werden, wobei der anorganische Füllstoff unter den anorganischen Nanopigmenten auf der Basis von Metalloxiden ausgewählt ist.

19. Zusammensetzung nach Anspruch 18, dadurch gekennzeichnet, daß es sich um eine Öl-in-Wasser-Dispersion oder eine Wasser-in-Öl-Dispersion handelt, die aus einer Fettphase und einer wässerigen Phase besteht.

20. Zusammensetzung nach einem der Ansprüche 18 bis 19, dadurch gekennzeichnet, daß es sich um eine Öl-in-Wasser-Emulsion oder eine Wasser-in-Öl-Emulsion handelt.

21. Zusammensetzung nach einem der Ansprüche 18 bis 20, dadurch gekennzeichnet, daß die anorganischen Nanopigmente unter den Titandioxiden, Zinkoxiden, Eisenoxiden, Zirconiumdioxiden und Cerdioxiden oder den Gemischen dieser Oxide ausgewählt sind.

22. Zusammensetzung nach einem der Ansprüche 18 bis 21, dadurch gekennzeichnet, daß die mittlere Größe der Primärpartikel der Metalloxide im Bereich von 5 bis 100 nm und vorzugsweise von 10 bis 50 nm liegt.

23. Zusammensetzung nach einem der Ansprüche 18 bis 22, dadurch gekennzeichnet, daß die anorganischen Nanopigmente unter den gegebenenfalls umhüllten Titandioxiden ausgewählt sind.

24. Zusammensetzung nach einem der Ansprüche 18 bis 23, dadurch gekennzeichnet, daß die Dispersion ferner mindestens einen weiteren Füllstoff umfaßt, der unter den verschiedenen anorganischen Füllstoffen von Metalloxid-Nanopigmenten und den organischen Füllstoffen ausgewählt ist.

25. Zusammensetzung nach Anspruch 24, dadurch gekennzeichnet, daß der weitere Füllstoff ausgewählt ist unter den Talken oder hydratisierten Magnesiumsilicaten, Glimmern oder Aluminosilicaten, Tonen, wie den Sericiten, Kaolin oder hydratisiertem Aluminiumsilicat, Bornitriden, Titandioxidglimmern, Pulvern aus Tetrafluorethylenpolymeren, Lauroyllysin, gefälltem Calciumcarbonat, Magnesiumcarbonat und Magnesiumhydrogencarbonat, nichtporösem kugelförmigem Siliciumdioxid, Hydroxylapatit, offenporigen oder hohlen Mikrokugeln aus Siliciumdioxid, die gegebenenfalls mit einem kosmetischen Wirkstoff imprägniert sind, Mikrokapseln aus Glas oder Keramik, Metallseifen, die von organischen Carbonsäuren mit 8 bis 22 Kohlenstoffatomen abgeleitet sind, wie Zinkstearat, Magnesiumstearat oder Lithiumstearat, Zinklaurat und Magnesiummyristat, Pulvern von nichtexpandierten synthetischen Polymeren, Pulvern von gegebenenfalls vernetzten kugelförmigen synthetischen Polymeren, Pulvern von organischen Materialien natürlicher Herkunft, mikroporösen Polymermikrokugeln, die gegebenenfalls mit kosmetischen Wirkstoffen imprägniert sind, Mikrokapseln aus gegebenenfalls vernetzten Polymeren, organischen Pigmenten, anorganischen Pigmenten, Perlglanzpigmenten und deren Gemischen.

26. Zusammensetzung nach einem der Ansprüche 18 bis 25, dadurch gekennzeichnet, daß sie 0,1 bis 70 Gew.-% Füllstoff(e), bezogen auf das Gesamtgewicht der Zusammensetzung, enthält.

27. Zusammensetzung nach einem der Ansprüche 18 bis 26, dadurch gekennzeichnet, daß sie ferner mindestens einen Emulgator enthält.

28. Zusammensetzung nach Anspruch 27, dadurch gekennzeichnet, daß der Mengenanteil des oder der Emulgatoren im Bereich von 0,5 bis 40 Gew.-%, vorzugsweise von 2 bis 15 Gew.-%, bezogen auf das Gesamtgewicht der Zusammensetzung, liegt.

29. Zusammensetzung nach einem der Ansprüche 18 bis 28, dadurch gekennzeichnet, daß sie ferner mindestens einen Hilfsstoff enthält, der ausgewählt ist unter den ionischen oder nichtionischen Verdickungsmitteln, herkömmlichen hydrophilen oder lipophilen organischen Sonnenschutzfiltern, die im UV-A-Bereich und/oder UV-B-Bereich wirksam sind, reizlindernden Mitteln, Antioxidantien, Trübungsmitteln, Stabilisatoren, Emollentien, Hydratisierungsmitteln, Vitaminen, Parfums, Konservierungsmitteln, Maskierungsmitteln, Färbemitteln, Weichmachern oder beliebigen weiteren üblicherweise in der Kosmetik verwendeten Inhaltsstoffen.

30. Zusammensetzung nach einem der Ansprüche 20 bis 29, dadurch gekennzeichnet, daß sie eine Wasser-in-Öl-Emulsion ist und die folgenden Eigenschaften aufweist:
(i) Wässerige Phase: 5 bis 70 Gew.-%, insbesondere 10 bis 50 Gew.-%, bezogen auf die gesamte Formulierung und
(ii) Ölphase: 30 bis 95 Gew.-%, insbesondere 40 bis 85 Gew.-%, bezogen auf die gesamte Formulierung.

31. Zusammensetzung nach einem der Ansprüche 20 bis 29, dadurch gekennzeichnet, daß sie eine Öl-in-Wasser-Emulsion ist und die folgenden Eigenschaften aufweist:
(i) Wässerige Phase: 5 bis 95 Gew.-%, insbesondere 10 bis 85 Gew.-%, bezogen auf die gesamte Formulierung und
(ii) Ölphase: 1 bis 50 Gew.-%, insbesondere 5 bis 30 Gew.-%, bezogen auf die gesamte Formulierung.

32. Verwendung der Zusammensetzung nach einem der Ansprüche 18 bis 31 als Grundlage von Pflegeprodukten und/oder Schminkprodukten und/oder Hygieneprodukten.

33. Verwendung der Zusammensetzung nach einem der Ansprüche 18 bis 31 als Grundlage von Produkten zum Schutz der Haut und/oder der Haare und/oder der Kopfhaut und/oder der Nägel und/oder der Wimpern und/oder der Augenbrauen und/oder der Schleimhäute gegen die Wirkungen der ultravioletten Strahlung.

34. Verfahren zur kosmetischen Behandlung der Haut und/oder der Haare und/oder der Kopfhaut und/oder der Nägel und/oder der Wimpern und/oder der Augenbrauen und/oder der Schleimhäute, dadurch gekennzeichnet, daß auf diese Träger eine nach einem der Ansprüche 18 bis 33 definierte Zusammensetzung aufgetragen wird.

35. Kosmetisches Behandlungsverfahren zum Schutz der Haut und/oder der Haare und/oder der Kopfhaut und/oder der Nägel und/oder der Wimpern und/oder der Augenbrauen und/oder der Schleimhäute gegen die Wirkungen der ultravioletten Strahlung, dadurch gekennzeichnet, daß auf diese Träger eine nach einem der Ansprüche 18 bis 33 definierte Zusammensetzung aufgetragen wird.
